# EUROPÄISCHE PATENTSCHRIFT

(11) **EP 0 991 467 B1**
(45) Veröffentlichungstag und Bekanntmachung des Hinweises auf die Patenterteilung: **02.05.2001**
(21) Anmeldenummer: 98937499.6
(22) Anmeldetag: 18.06.1998
(51) Int. Cl.: B01J 23/36, B01J 21/02, C07C 6/06, B01J 31/12

(54) **TRÄGERKATALYSATOR ZUR VERWENDUNG BEI DER HERSTELLUNG VON CYCLOALKADIENEN IN EINER METATHESEREAKTION**
SUPPORTED CATALYST USED IN THE PRODUCTION OF CYCLOALKADIENES IN A METATHESIS REACTION
CATALYSEUR SUR SUPPORT UTILISABLE DANS LA FABRICATION DE CYCLOALCADIENES DANS UNE REACTION DE METATHESE

(30) Priorität: 26.06.1997 DE 19727256
(43) Veröffentlichungstag der Anmeldung: 12.04.2000
(73) Patentinhaber: Consortium für elektrochemische Industrie GmbH, 81379 München (DE)
(72) Erfinder: EBERLE, Hans-Juergen, D-81477 München (DE); ZEITLER, Norbert, D-80339 München (DE); CSELLICH, Christine, D-82008 Unterhaching (DE)
(74) Vertreter: Schuderer, Michael, Dr.
(86) Internationale Anmeldenummer: EP9803689
(87) Internationale Veröffentlichungsnummer: WO9900189

(56) Entgegenhaltungen:
- EP-A- 0 475 245
- GB-A- 1 377 161
- US-A- 3 892 817
- US-A- 5 183 791
- X.XIAODING, C.BOELHOUVER, J.I.BENECKE, D.VONK,J.C.MOL: "Re2O7/Al2O3.B2O3 Metathesis Catalyst" J.CHEM.SOC., FARADAY TRANS. 1, Bd. 82, 1986, Seiten 1945-1953, XP002082478
- PATENT ABSTRACTS OF JAPAN vol. 096, no. 011, 29. November 1996 & JP 08 176022 A (SUMITOMO CHEM CO LTD), 9. Juli 1996

## Beschreibung

Die Erfindung betrifft einen Trägerkatalysator zur Verwendung bei der Herstellung von Cycloalkadienen in einer Metathesereaktion sowie ein Verfahren zur Herstellung von Cycloalkadienen in Flüssigphase durch Metathesereaktion von Cycloalkenen, Cyclopolyenen, linearen Polyenen sowie deren Gemischen in Gegenwart des Trägerkatalysators.

Cycloalkadiene mit einer Ringgröße zwischen 12 und 18 C-Atomen werden unter anderem bei der Herstellung von makrocyclischen Oxo-Verbindungen eingesetzt. Besonderes Interesse gilt hierbei den makrocyclischen Ketonen und Epoxiden, die als wertvolle Moschusriechstoffe Verwendung finden. Ein weiteres Einsatzgebiet dieser makrocyclischen Oxo-Verbindungen ist die Verwendung als selektive Komplexbildner bei der Herstellung von gamma-Cyclodextrin.

Aus der GB-A 1105565 ist bekannt, Cycloalkadiene wie Cyclohexadecadien durch Metathesereaktion von Cycloocten an einem Re₂O₇/γ-Al₂O₃-Kontakt herzustellen. Der dem genannten Verfahren zugrundeliegende Reaktionstyp kann als Dimerisierung des Ausgangsprodukts beschrieben werden. Die Ausbeuten werden mit 6 Gew%, bezogen auf eingesetztes Cycloocten, angegeben. Die unbefriedigende Ausbeute ist darauf zurückzuführen, daß die Reaktion in bezug auf die Dimerisierung unbefriedigend verläuft und zum größten Teil höhere Oligomere bzw. Polymere, die cyclischer wie acyclischer Natur sein können, gebildet werden.

Aus der EP-A 182333 (US-A 4668836) ist bekannt, daß die Selektivität der Metathesereaktion zu dem gewünschten Dimer deutlich erhöht werden kann, wenn die Reaktion bei hohen Verdünnungen ausgeführt wird. Unter Verwendung des Katalysatorsystems Re₂O₇/γ-Al₂O₃/SnR₄, wobei R für einen Alkylrest steht, lassen sich Selektivitäten von bis zu 50 Mol% erhalten, wenn mit verdünnten, 0.01- bis 0.05-molaren Cycloolefinlösungen in einem Metathese-inerten Lösungsmittel gearbeitet wird. Die bevorzugten Reaktionstemperaturen liegen im Bereich von 0°C bis 50°C. Nach der EP-B 343437 lassen sich bei der Herstellung von Cycloalkadienen durch Metathesereaktion in Gegenwart eines Re₂O₇/Al₂O₃/SnR₄-Katalysators auch oligomere und polymere Cycloolefine sowie deren Gemische mit entsprechenden monomeren Cycloolefinen als Ausgangsprodukte einsetzen.
Das in der EP-A 182333 und in der EP-B 343437 beschriebene Katalysatorsystem hat jedoch den Nachteil, daß es bei Temperaturen von über 50°C sehr schnell desaktiviert wird. Reaktionstemperaturen von über 50°C wären aber von großem Vorteil, da sich damit die Produktivität des Verfahrens, das heißt die Raum-Zeit-Leistung, deutlich steigern ließe.

Es bestand daher die Aufgabe einen Trägerkatalysator und ein Verfahren zur Verfügung zu stellen, mit dem die Herstellung von Cycloalkadienen durch Metathesereaktion auch bei höheren Reaktionstemperaturen mit hoher Selektivität erfolgt.

Gegenstand der Erfindung ist ein Trägerkatalysator zur Verwendung bei der Herstellung von Cycloalkadienen in einer Metathesereaktion enthaltend
a) γ-Al₂O₃ als Trägermaterial,
b) 2 bis 20 Gew.-% Re₂O₇,
c) 0.5 bis 5 Gew.-% B₂O₃,
d) 1 bis 20 Gew.-% SnR₄ oder SnO₂ oder eines Gemisches von SnR₄ und SnO₂, wobei R für einen Alkyl- oder Arylrest steht,
und die Angaben in Gew.-% jeweils auf γ-Al₂O₃ bezogen sind.

Als Trägerkatalysatoren werden solche eingesetzt, welche als Trägermaterial γ-Al₂O₃ mit einer spezifischen Oberfläche von 100 bis 300 m²/g nach BET (Bestimmungsmethode nach Brunauer, Emmett und Teller) aufweisen. Das Trägermaterial wird als Formkörper, vorzugsweise in Form von Hohlsträngen, Kugeln, Zylindern, Würfeln und Kegeln eingesetzt.

Der Gewichtsanteil an Re₂O₇ beträgt vorzugsweise 2 bis 10 Gew.-%, bezogen auf den Aluminiumoxid-Anteil.

Wesentlich für die Verlängerung der Standzeit des Trägerkatalysators bei höheren Reaktionstemperaturen ist dessen Dotierung mit Boroxid. Der Gehalt an Boroxid beträgt 0.5 bis 5 Gew.-%, bezogen auf den Aluminiumoxid-Anteil.

Erfindungsgemäß wird der Katalysator mit den unter d) genannten Zinnverbindungen dotiert. Überraschenderweise wurde festgestellt, daß sich in Anwesenheit von Komponente d), die durch die Boroxid-Dotierung erhaltene Verlängerung der Standzeit des Trägerkatalysators weiter steigern läßt. Als Komponente d) geeignet sind SnR₄-Verbindungen bei denen R für gleiche oder verschiedene Substituenten aus der Gruppe C₁- bis C₆-Alkylrest und Phenylrest steht. Beispiele hierfür sind Zinntetramethyl, Zinntetraethyl, Zinn-tetra-n-butyl. Als Komponente d) bevorzugt werden Gemische aus den genannten SnR₄-Verbindungen mit SnO₂ oder SnO₂ als alleinige Komponente d). Der Gehalt an Komponente d) in der Trägerkatalysator-Zusammensetzung beträgt vorzugsweise 1 bis 20 Gew.-%, besonders bevorzugt 1 bis 10 Gew.-%, bezogen auf den Aluminiumoxid-Anteil.

Die Herstellung des Katalysators erfolgt mit dem Fachmann bekannten Verfahrensweisen. Üblicherweise wird der Aluminiumoxid-Träger mit wässrigen Lösungen einer wasserlöslichen Rhenium-Verbindung, beispielsweise Ammoniumperrhenat, und einer wasserlöslichen Borverbindung, beispielsweise Borsäure, imprägniert. Es kann auch ein Gemisch aus Wasser und einem mit Wasser mischbaren organischen Lösungsmittel, beispielsweise Dioxan, zur Lösung der Verbindungen verwendet werden. Die Reihenfolge der Imprägnierung mit den wässrigen Lösungen der Rheniumverbindung bzw. der Borverbindung ist dabei beliebig. Vorzugsweise wird so vorgegangen, daß die Rhenium- und die Borverbindung in einer Imprägnierlösung in einem Schritt auf den Aluminiumoxid-Träger aufgetragen werden. Zur Imprägnierung wird das wässrige Gemisch aus Trägerkomponente, Rhenium- und Borverbindung mehrere Stunden, im allgemeinen 6 bis 12 Stunden, auf Siedetemperatur erhitzt und die flüssige Phase, beispielsweise durch Abdekantieren, entfernt. Der imprägnierte Träger wird anschließend getrocknet.

Zur Aktivierung des Katalysators, das heißt zur Umwandlung der Rhenium- bzw. Borverbindung in die Aktivkomponenten b) und c), wird der imprägnierte Träger in sauerstoffhaltiger Atmosphäre, im allgemeinen Luft, für 1 bis 3 Stunden auf 500°C bis 600°C erhitzt. Nach Abschluß der Calcinierung wird der Träger weitere 1 bis 3 Stunden unter Inertgas, beispielsweise Stickstoff oder Argon, erhitzt und in Inertgas-Atmosphäre auf Raumtemperatur abgekühlt.

Zur Dotierung mit einer SnR₄-Verbindung wird so vorgegangen, daß der Trägerkatalysator in einem weiteren Schritt mit einer Lösung der entsprechenden Zinnverbindung in einem organischen Lösungsmittel, beispielsweise Petrolether, imprägniert wird. Dazu kann ein Gemisch aus Trägerkatalysator und der Lösung der Zinnverbindung mehrere Stunden gerührt werden, analog der obengenannten Vorgehensweise zur Imprägnierung. Es kann auch so vorgegangen werden, daß der Trägerkatalysator unmittelbar vor der Metathesereaktion imprägniert wird. Dazu wird der Reaktor mit Trägerkatalysator befüllt und es wird mehrere Stunden eine Lösung der Zinnverbindung im Reaktor umgepumpt.

Nach der Verwendung bei der Metathesereaktion kann der Trägerkatalysator regeneriert werden und erneut eingesetzt werden. Zur Regenerierung wird der Katalysator aus dem Metathesereaktor entfernt und mit einem inerten organischen Lösungsmittel gewaschen, beispielsweise mit Toluol, Cyclohexan oder Methylenchlorid. Danach wird der Trägerkatalysator getrocknet und einer Calcinierung analog der oben, zur Aktivierung des Katalysators, beschriebenen unterzogen. Dazu wird der Trägerkatalysator in sauerstoffhaltiger Atmosphäre, im allgemeinen Luft, für 1 bis 3 Stunden auf 500°C bis 600°C erhitzt. Dabei ist darauf zu achten, daß im Katalysator eine Temperatur von 600°C nicht überschritten wird. Gegebenenfalls muß der Sauerstoffgehalt der Regenerierluft durch Zugabe eines Inertgases, beispielsweise Stickstoff oder Argon, vermindert werden. Nach erfolgter oxidativer Regenerierung wird der Träger weitere 1 bis 3 Stunden unter Inertgas, beispielsweise Stickstoff oder Argon, erhitzt und in Inertgas-Atmosphäre auf Raumtemperatur abgekühlt. Bei der Regenerierung kommt es zu keiner Änderung des Gehalts an R₂O₇ bzw. B₂O₃; die SnR4-Komponente wird zu SnO₂ umgewandelt. Bei der Wiederverwendung eines regenerierten, ursprünglich mit Zinnalkyl dotierten Trägerkatalysators wurde nun überraschenderweise festgestellt, daß sich die mit der Boroxid-Dotierung erhaltene erhöhte Standzeit nach der Regenerierung deutlich verbessert. Dieser Effekt dürfte auf eine synergetische Wechselwirkung zwischen Boroxid- und Zinnoxid-Komponente zurückzuführen sein.

Zur Dotierung mit SnO₂ kann alternativ zur Regenerierung eines Zinnalkyl-haltigen Trägerkatalysators so vorgegangen werden, daß der Aluminiumoxid-Träger, analog der Vorgehensweise bei der Imprägnierung mit einer Rhenium- bzw. Borverbindung, mit einem wasserlöslichen Zinnsalz, beispielsweise Zinn(IV)chlorid, imprägniert wird.

Ein weiterer Gegenstand der Erfindung ist ein Verfahren zur Herstellung von Cycloalkadienen in Flüssigphase durch Metathesereaktion von Cycloalkenen, Cyclopolyenen, linearen Polyenen sowie deren Gemischen in Gegenwart eines Trägerkatalysators bei einer Reaktionstemperatur von 0°C bis 100°C, dadurch gekennzeichnet, daß der Trägerkatalysator
a) γ-Al₂O₃ als Trägermaterial,
b) 2 bis 20 Gew.-% Re₂O₇,
c) 0.5 bis 5 Gew.-% B₂O₃,
d) 1 bis 20 Gew.-% SnR₄ oder SnO₂ oder eines Gemisches von SnR₄ und SnO₂, wobei R für einen Alkyl- oder Arylrest steht, enthält, und die Angaben in Gew.-% jeweils auf γ-Al₂O₃ bezogen sind.

Geeignete Edukte sind aus der Gruppe der Cycloolefine solche mit einer Ringgröße von 4 bis 12 C-Atomen. Bevorzugt werden Cyclopenten, Cyclohepten, Cycloocten, Cyclooctadien, Cyclodecen und Cyclododecen. Besonders bevorzugt werden Cyclohepten und Cycloocten eingesetzt. Geeignete Cyclopolyene oder lineare Polyene sind solche, welche mit den genannten Cycloolefinen, beispielsweise als Nebenprodukte bei metathetischen Dimerisierungen, erhalten werden. Im allgemeinen weisen die Cyclopolyene und die linearen Polyene einen Polymerisationsgrad von 3 bis 20 auf. Es können auch Gemische aus zwei oder mehreren Verbindungen aus der Gruppe umfassend Cycloolefine, Cyclopolyene und lineare Polyene eingesetzt werden.

Die Edukte werden in Form einer 0.01- bis 0.1-molaren, vorzugsweise 0.03- bis 0.06-molaren, Lösung in einem inerten organischen Lösungsmittel eingesetzt. Geeignete Lösungsmittel sind beispielsweise Pentan, Hexan, Heptan, Cyclopentan, Cyclohexan, Petrolether, Methylenchlorid, Chloroform und Tetrachlorkohlenstoff. Die Reaktion kann prinzipiell bei Temperaturen zwischen 0°C und 100°C durchgeführt werden. Mit dem erfindungsgemäßen Trägerkatalysator steht allerdings erstmals ein Metathesekatalysator zur Verfügung, der auch bei höheren Reaktionstemperaturen, mit entsprechend verbesserter Raum-Zeit-Leistung, die in der Praxis erforderlichen Standzeiten aufweist. Für die Reaktionstemperatur ist daher der Bereich von 50°C bis 100°C stark bevorzugt, insbesondere der Bereich von 60°C bis 100°C. Beim Einsatz von Lösungsmitteln, deren Siedepunkt unterhalb der Reaktionstemperatur liegt, kann auch unter Druck gearbeitet werden. Im allgemeinen beträgt der Druck von 1 bar abs. bis 10 bar abs..

Die Metathesereaktion wird im allgemeinen in einem Festbettreaktor, beispielsweise einem, mit Trägerkatalysator befüllten, senkrecht stehenden Rohrreaktor durchgeführt, durch den die Lösung der Edukte in vertikaler Richtung strömt. Die Strömungsgeschwindigkeit wird dabei so eingestellt, daß Verweilzeiten von 10 bis 600 Sekunden, vorzugsweise 10 bis 200 Sekunden, resultieren. Das den Reaktor verlassende Reaktionsgemisch wird einer Destillationsvorrichtung aufgegeben und in seine Komponenten getrennt. Das Zielprodukt fällt als relativ hochsiedende Fraktion an. In der Praxis wird zumeist ein kontinuierlicher Betrieb aufrechterhalten, wobei das Lösungsmittel sowie gegebenenfalls nicht umgesetztes Ausgangsprodukt im Kreislauf geführt und nach Beschickung mit neuem Ausgangsprodukt in den Reaktor zurückgeleitet wird. Bei der kontinuierlichen Fahrweise kann die Verdampfung/Kondensation des Lösungsmittelgemisches in konventioneller Weise (z.B. Verdampfer betrieben mit Dampf, Kondensator betrieben mit Kühlmedien) durchgeführt werden. Vorteilhaft ist jedoch der Einsatz von energetisch günstigeren Verfahren wie beispielsweise die Brüdenkompression, da hiermit der Energieaufwand deutlich gesenkt und damit das Verfahren wirtschaftlicher durchgeführt werden kann.

Die nachfolgenden Beispiele dienen der weiteren Erläuterung der Erfindung:

### Beispiel 1 (Vergleichsbeispiel):

### Herstellung des Vergleichskatalysator (γ-Al₂O₃/Re₂O₇/Sn(CH₃)₄)

NH₄ReO₄ (5.5 Gew% bezogen auf Al₂O₃) wurde in einem Dioxan/Wasser-Gemisch (80/20 v/v) aufgelöst und das γ-Al₂O₃ in Form von Strangpresslingen mit einer Länge von 3 bis 8 mm, einem Durchmesser von 1.5 bis 1.8 mm und einer BET-Oberfläche von 190 m²/g dazugegeben. Anschließend wurde das Gemisch zum Sieden erhitzt. Nach einer Imprägnierdauer von ca. 10 Stunden wurde die flüssige Phase abdekantiert und der Katalysator bei 120°C vorgetrocknet. Die Aktivierung erfolgte durch eine 2-stündige Luftbehandlung zwischen 500°C und 600°C. Danach wurde unter Beibehaltung der Temperatur die Luft durch Stickstoff ersetzt. Nach weiteren 2 Stunden wurde auf Raumtemperatur abgekühlt, wobei darauf geachtet wurde, daß kein Sauerstoff an den Katalysator gelangte. Der Gehalt an Re₂O₇ betrug 3.7 Gew%. Der Trägerkatalysator wurde dann mit einer Pentanlösung von Sn(CH₃)₄ behandelt, wobei die Imprägnierung direkt im Metathesereaktor erfolgte. Der Gehalt an Sn(CH₃)₄ betrug 2.5 Gew%. Die Angaben in Gew% beziehen sich jeweils auf Aluminiumoxid.

### Beispiel 2:

### Herstellung eines Boroxid-haltigen Katalysators (γ-Al₂O₃/Re₂O₇/B₂O₃/Sn(CH₃)₄)

Borsäure (11.7 Gew% bezogen auf Al₂O₃) wurde in Wasser gelöst. Anschließend wurde in dieser Lösung NH₄ReO₄ (5.5 Gew% bezogen auf Al₂O₃) aufgelöst. Zu dieser Lösung wurde nun Dioxan bis zur Einstellung eines Verhältnisses Dioxan/Wasser von 85/15 v/v) zugegeben. Anschließend wurden die Al₂O₃-Strangpresslinge analog Beispiel 1 zugegeben, und die Imprägnierung, Calcinierung und Sn(CH₃)₄-Behandlung analog Beispiel 1 durchgeführt. Der Gehalt an Re₂O₇ betrug 3.7 Gew%, der Boroxid-Gehalt 1.8 Gew%, der Sn(CH₃)₄-Gehalt war 2.5 Gew%. Die Angaben in Gew% beziehen sich jeweils auf Aluminiumoxid.

### Beispiel 3 (Vergleichsbeispiel) :

### Metathese eines Cyclopolyoctenylengemisches mit dem boroxidfreien Katalysator aus Beispiel 1 bei 60°C

Ein beheizbarer Reaktor in vertikaler Anordnung (Länge 500 mm, Innendurchmesser 26.5 mm) wurde mit 200 g Katalysator aus Beispiel 1 befüllt. Anschließend wurde der Reaktor auf 60°C aufgeheizt und durch das Katalysatorbett eine 0.048-molare Lösung (Konzentrationsangabe bezogen auf die Monoolefineinheit Cycloocten) eines Cyclopolyoctenylengemisches in Pentan geleitet. Der Druck im Reaktor wurde auf 6 bar abs. eingestellt, um ein Sieden von Pentan zu verhindern. Die Zusammensetzung des Cyclopolyoctenylengemisches ist in Tabelle 1 angegeben:

**Tabelle 1:**

| Polymerisationsgrad | Anteil (Gew%) |
|---|---|
| 3 | 42.5 |
| 4 | 23.8 |
| 5 | 13.3 |
| 6 | 7.9 |
| 7 und größer | 10.1 |

Die mittlere Verweilzeit des Gemisches im Reaktor war 80 Sekunden. Das den Reaktor verlassende Reaktionsgemisch wurde einer Destillationsvorrichtung zugeführt, wobei das n-Pentan abdestilliert und nach Beladen mit frischem Cyclopolyoctenylengemisch wieder dem Reaktor zugeführt wurde. In der Tabelle 2 sind die durchschnittlichen Konzentrationen von Cyclohexadecadien-1.9 (CHDD-Konzentrationen) im Reaktionsgemisch, in Abhängigkeit von der Versuchsdauer, aufgeführt. Die Probennahme erfolgte direkt nach dem Reaktor.

**Tabelle 2:**

| Versuchsdauer (h) | CHDD-Gehalt (g/l) |
|---|---|
| 1 | 1.1 |
| 60 | 1.0 |
| 110 | 0.94 |
| 160 | 0.86 |
| 230 | 0.69 |
| 290 | 0.59 |
| 330 | 0.54 |
| 360 | 0.46 |

### Beispiel 4:

### Metathese eines Cyclopolyoctenylengemisches mit dem boroxid-haltigen Katalysator aus Beispiel 2 bei 60°C

Es wurde analog Beispiel 3 vorgegangen, mit dem Unterschied, daß der in Beispiel 2 hergestellte Katalysator eingesetzt wurde.
In Tabelle 3 sind die gemessenen CHDD-Konzentrationen in Abhängigkeit von der Versuchsdauer aufgeführt.

**Tabelle 3:**

| Versuchsdauer (h) | CHDD-Gehalt (g/l) |
|---|---|
| 1 | 1.2 |
| 110 | 1.0 |
| 300 | 0.85 |
| 400 | 0.80 |
| 500 | 0.76 |
| 690 | 0.70 |
| 850 | 0.67 |
| 910 | 0.64 |

### Beispiel 5:

### Metathese eines Cyclopolyoctenylengemisches mit einem regeneriertem, Boroxid und Zinnoxid enthaltenden Katalysator bei 60°C

Der in Beispiel 4 eingesetzte Katalysator wurde aus dem Reaktor entfernt und mit Toluol gewaschen. Danach wurde der Katalysator bei 100°C getrocknet und 2 Stunden in Luftatmosphäre, weitere 2 Stunden unter Stickstoff bei jeweils 500°C bis 600°C calciniert und anschließend auf Raumtemperatur abgekühlt. Der Gehalt an Rheniumoxid und Boroxid blieb unverändert. Das Zinntetramethyl wurde in Zinnoxid umgewandelt. Der SnO₂-Gehalt betrug 0.53 Gew%, bezogen auf Aluminiumoxid.
Der so regenerierte Katalysator wurde anschließend mit einer Zinntetramethyl-Pentan-Lösung analog Beispiel 3 behandelt (2.5 Gew% Sn(CH₃)₄) und zur Metathese eines Cyclooctenylengemisches analog Beispiel 3 eingesetzt.
In Tabelle 4 sind die CHDD-Konzentrationen in Abhängigkeit von der Versuchsdauer aufgeführt.

**Tabelle 4:**

| Versuchsdauer (h) | CHDD-Gehalt (g/l) |
|---|---|
| 1 | 1.3 |
| 60 | 1.2 |
| 200 | 1.1 |
| 300 | 1.1 |
| 450 | 1.1 |
| 600 | 1.1 |
| 700 | 1.1 |
| 850 | 1.1 |
| 970 | 1.1 |
| 1150 | 1.0 |

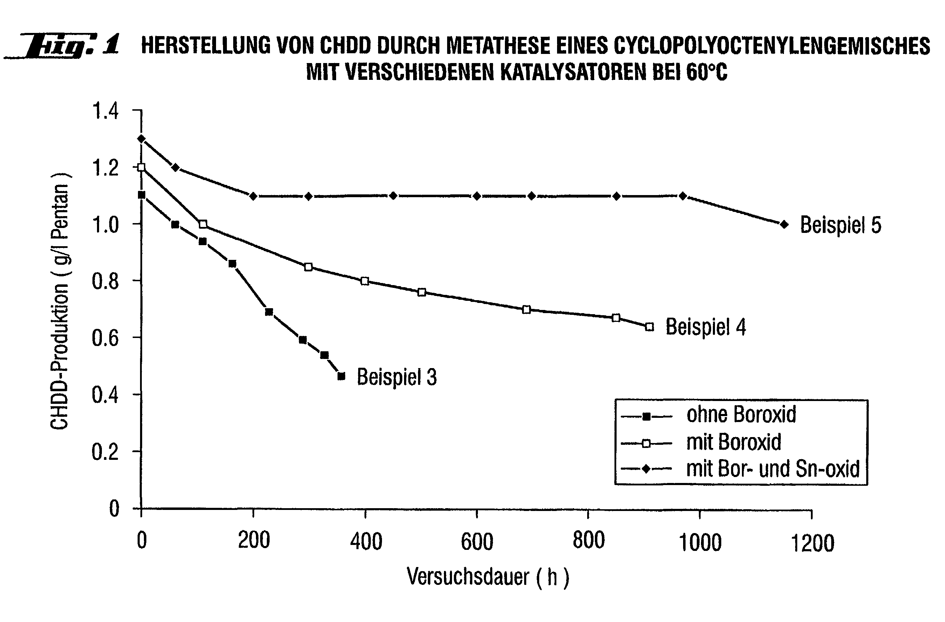

Die Ergebnisse aus den Metathesereaktionen gemäß Vergleichsbeispiel 3 (Tabelle 2) und gemäß der erfindungsgemäßen Beispielen 4 (Tabelle 3) und 5 (Tabelle 4) sind in der Abbildung 1 graphisch zusammengefaßt. Es ist deutlich zu sehen, daß gegenüber herkömmlichen Metathesekatalysatoren (Beispiel 3: Al₂O₃/Re₂O₇/Sn(CH₃)₄) bei Boroxid-Dotierung (Beispiel 4) deutlich höhere Standzeiten resultieren, wobei sich diese mittels SnO₂-Dotierung (Beispiel 5) noch weiter steigern lassen.

## Patentansprüche

1. Trägerkatalysator zur Verwendung bei der Herstellung von Cycloalkadienen in einer Metathesereaktion enthaltend
a) γ-Al₂O₃ als Trägermaterial,
b) 2 bis 20 Gew.-% Re₂O₇,
c) 0.5 bis 5 Gew.-% B₂O₃,
d) 1 bis 20 Gew.-% SnR₄ oder SnO₂ oder eines Gemisches von SnR₄ und SnO₂, wobei R für einen Alkyl- oder Arylrest steht, und die Angaben in Gew.-% jeweils auf γ-Al₂O₃ bezogen sind.

2. Trägerkatalysator gemäß Anspruch 1, dadurch gekennzeichnet, daß 1 bis 10 Gew.-%, bezogen auf den Aluminiumoxid-Anteil, SnO₂, gegebenenfalls im Gemisch mit SnR₄-Verbindungen, bei denen R für gleiche oder verschiedene Substituenten aus der Gruppe C₁- bis C₆-Alkyl- und Phenylrest steht, enthalten sind.

3. Trägerkatalysator gemäß Anspruch 1, dadurch gekennzeichnet, daß 1 bis 10 Gew.-%, bezogen auf den Aluminiumoxid-Anteil, eines Gemisches aus SnO₂ und SnR₄-Verbindung enthalten sind.

4. Verfahren zur Herstellung von Cycloalkadienen in Flüssigphase durch Metathesereaktion von Cycloalkenen, Cyclopolyenen, linearen Polyenen sowie deren Gemischen bei einer Reaktionstemperatur von 0°C bis 100°C in Gegenwart eines Trägerkatalysators gemäß Anspruch 1 bis 3.

5. Verfahren nach Anspruch 4, dadurch gekennzeichnet, daß die Reaktionstemperatur von 50°C bis 100°C und der Druck von 1 bar abs. bis 10 bar abs. beträgt.

## Claims

1. Supported catalyst for use in preparing cycloalkadienes in a metathesis reaction, comprising
a) γ-Al₂O₃ as support,
b) from 2 to 20% by weight of Re₂O₇,
c) from 0.5 to 5% by weight of B₂O₃, and
d) from 1 to 20% by weight of SnR₄ or SnO₂ or of a mixture of SnR4 and SnO₂, where R is an alkyl or aryl radical and the data in % by weight are in each case based on γ-Al₂O₃.

2. Supported catalyst according to Claim 1, characterized in that from 1 to 10% by weight, based on the aluminium oxide content, of SnO₂, if desired in a mixture with SnR₄ compounds, are present, in which R are identical or different substituents selected from the class consisting of C₁-C₆-alkyl and phenyl radicals.

3. Supported catalyst according to Claim 1, characterized in that from 1 to 10% by weight, based on the aluminium oxide content, of a mixture of SnO₂ and SnR4 compound are present.

4. Process for preparing cycloalkadienes in the liquid phase by a metathesis reaction of cycloalkenes, cyclopolyenes, linear polyenes, and also mixtures of these, at a reaction temperature of from 0 to 100°C in the presence of a supported catalyst according to Claims 1 to 3.

5. Process according to Claim 4, characterized in that the reaction temperature is from 50 to 100°C and the pressure is from 1 bar abs. to 10 bar abs.

## Revendications

1. Catalyseur support pour l'utilisation lors de la production de cycloalcadiènes dans une réaction de métathèse, contenant
a) γ-Al₂O₃ comme matériau support
b) 2 à 20% en poids de Re₂O₇,
c) 0,5 à 5% en poids de B₂O₃,
d) 1 à 20% en poids de SnR₄ ou de SnO₂ ou d'un mélange de SnR₄ et de SnO₂, R représentant un radical alkyle ou aryle, et les indications en % en poids se rapportant à chaque fois à γ-Al₂O₃.

2. Catalyseur support selon la revendication 1, caractérisé en ce que 1 à 10% en poids, par rapport à la proportion d'oxyde d'aluminium, de SnO₂, le cas échéant en mélange avec des composés SnR₄, dans lesquels R représente des substituants identiques ou différents parmi le groupe des radicaux alkyle en C₁ à C₆ et le radical phényle, sont contenus.

3. Catalyseur support selon la revendication 1, caractérisé en ce que 1 à 10% en poids, par rapport à la proportion d'oxyde d'aluminium, d'un mélange de SnO₂ et de composé SnR₄ sont contenus.

4. Procédé pour la préparation de cycloalcadiènes en phase liquide par réaction de métathèse de cycloalcènes, de cyclopolyènes, de polyènes linéaires ainsi que de leurs mélanges à une température de réaction de 0°C à 100°C en présence d'un catalyseur support selon les revendications 1 à 3.

5. Procédé selon la revendication 4, caractérisé en ce que la température de réaction est de 50°C à 100°C et la pression de 1 bar abs. à 10 bars abs.
